# EUROPEAN PATENT APPLICATION

(11) **EP 4 582 543 A1**
(43) Date of publication of application: **09.07.2025**
(21) Application number: 24863844.7
(22) Date of filing: 17.01.2024
(51) Int. Cl.: C12N 15/113, C12N 15/85, A61K 48/00, A61K 31/713, A61P 35/00

(54) **USE OF GENES PRDX5 AND GPX4 IN PREPARATION OF DRUG FOR CASTRATION-RESISTANT PROSTATE CANCER**

(30) Priority: 02.11.2023 CN 202311449093
(71) Applicant: Jiangnan University, Wuxi, Jiangsu 214122 (CN)
(72) Inventor: WANG, Rong, Wuxi, Jiangsu 214122 (CN); CHEN, Yongquan, Wuxi, Jiangsu 214122 (CN)
(74) Representative: Ipside
(86) International application number: PCT/CN2024/072783
(87) International publication number: WO 2025/091701

(57) **Abstract**

The present disclosure discloses application of PRDX5 and GPX4 genes in preparation of drugs for castration-resistant prostate cancer and belongs to the technical field of biological medicines. The present disclosure proposes that silencing or interfering with the expression of the PRDX5 and/or GPX4 genes can be used in the treatment or adjuvant treatment of castration-resistant prostate cancer. The present disclosure proposes for the first time a new strategy for preparing the drugs for the treatment of CRPC using siRNA in combination with an androgen receptor antagonist. The pharmaceutical composition comprising siRNA in combination with an androgen receptor antagonist according to the present disclosure can be used for the treatment of castration-resistant prostate cancer and significantly improves the inhibitory effect of enzalutamide on castration-resistant prostate cancer, which provides the basis for the design and clinical application of nucleic acid drugs with important clinical therapeutic significance.

## Description

### Technical Field

The present disclosure belongs to the technical field of biological medicines and specifically relates to application of PRDX5 and GPX4 genes in preparation of drugs for a castration-resistant prostate cancer.

### Background of the Invention

Androgen deprivation therapy is the standard of care for an advanced prostate cancer. However, patients eventually progress to a castration-resistant prostate cancer (CRPC) after an average of 1-3 years of treatment. CRPC is defined as a prostate cancer that has still progressed after initial androgen deprivation therapy (ADT). Since docetaxel was shown to prolong the overall survival of patients with a metastatic castration-resistant prostate cancer (mCRPC) in 2004, drugs targeting the disease stage of mCRPC, such as abiraterone acetate, enzalutamide, and cabazitaxel, have emerged in recent years and have changed the current status of treatment for these patients. However, it is difficult to completely reverse CRPC. Therefore, the search for other effective therapeutic targets is another research hotspot in the treatment of CRPC.

Peroxiredoxin 5 (PRDX5), as the only atypical 2-Cys peroxiredoxin in mammals, is localized in mitochondria, peroxisomes, cytoplasm and nucleus, and is a cytoprotective antioxidant enzyme against endogenous or exogenous peroxidative attack. It has been shown that PRDX5 is essential for cancer cell viability under conditions of oxidative stress (OS), and overexpression of PRDX5 helps to protect cells from OS-induced apoptosis. OS refers to a state of imbalance between oxidative and antioxidant effects *in vivo,* and is the result of an imbalance between two opposing forces of reactive oxygen species (ROS) generation and antioxidant effects, which can promote pathologic defects in organisms such as cancers and diabetes and damage cellular components such as DNA. ROS are very reactive oxygen species, including hydrogen peroxide (H₂O₂), superoxide anion (O²⁻), and hydroxyl radicals, etc. Cancer cells express elevated levels of ROS. However, antioxidants such as PRDX5 present in cancer cells can detoxify ROS, which promotes the growth and development of cancer cells. When the intercellular balance between ROS and antioxidant effects is disrupted, the intracellular ROS threshold level is elevated, leading to OS-mediated apoptosis.

Phospholipid hydroperoxide glutathione peroxidase (GPX4) is an essential antioxidant peroxidase that directly reduces phospholipid hydroperoxides even though they are incorporated into membranes and lipoproteins. It also reduces fatty acid hydroperoxides, cholesterol hydroperoxides and thymine hydroperoxides. The prevention of membrane lipid peroxidation plays a key role in protecting cells from oxidative damage. A paper published in Nature in 2017 reported that cancer cells in a high mesenchymal therapy-resistant cell state are selectively sensitive to ferroptosis, a non-apoptotic cell death caused by iron-dependent accumulation of lipid reactive oxygen species, which can be induced by inhibiting the lipid hydroperoxidase GPX4. It is found that sensitivity to GPX4 inhibition is susceptibility of general persistent cells.

Small nucleic acid drugs are pharmacologically effective through RNA interference (RNAi), which provides a limited selection of the appropriate target with high specificity and allows for the expansion of the drug target to the upstream RNA of functional proteins to regulate target gene expression at the post-transcriptional level. In the early stages of developing siRNA therapeutics, many drugs are designed based on completely unmodified or slightly modified siRNAs to reach appropriate tissues and then silence a target gene. These molecules can mediate gene silencing *in vivo.* However, limited efficacy and potential off-target effects may be observed with these approaches. Therefore, chemically modified siRNAs, such as replacing 2'-OH with a 2'-O-methyl (2'-OMe) or 2'-methoxyethyl (2'-MOE) group, can effectively inhibit innate immune activation driven by immune stimulation of siRNA, enhance activity and specificity, and reduce off-target-induced toxicity. Many chemical modifications have been established and tested in order to increase the potency of siRNAs and reduce their potential toxicity. The present disclosure intends to prepare the found effective therapeutic targets for CRPC, PRDX5 and GPX4, into siRNA drugs with modifications that have an RNA interference effect in cells and mice, so as to reduce the expression of PRDX5, GPX4, and PRDX5+GPX4 and retard the development of CRPC.

### Summary of the Invention

The technical problem to be solved by the present disclosure is to find an effective therapeutic target to provide a nucleic acid drug, i.e., the nucleic acid drug siPRDX5, which is effective in treating CRPC, achieving a significant enhancement of the therapeutic efficacy against CRPC.

The present disclosure has found a nucleic acid drug, i.e., siPRDX5 and siGPX4, for the treatment of CRPC after a lot of research and exploration. The results of the study show that by administrating to a human prostate cancer LNCaP cells, mouse MyC-CaP cells, and drug-tolerant persister (DTP) cells formed by LNCaP and MyC-CaP cells, either siPRDX5 or siGPX4 alone can reduce the expression of PRDX5 or GPX4 in cancer cells, and the combination of siPRDX5+siGPX4 can reduce the expression of both PRDX5 and GPX4. Gene expression and protein expression are determined by q-RTPCR and Western Blot assays.

In the present disclosure, siPRDX5 or siGPX4 or siPRDX5+siGPX4 are administrated to LNCaP/MyC-CaP-DTP and 22Rv1 cells, the drug effects are determined by CCK8 cell proliferation assay, and the results show that siPRDX5 or siGPX4 or siPRDX5+siGPX4 can significantly inhibit the growth of LNCaP-DTP, MyC-CaP-DTP and 22Rv1 cells. Meanwhile, a CRPC mouse model formed by continuous gavage of ENZ in *C-MYC*-overexpressing prostate cancer mice is constructed. siPRDX5 or siGPX4 or siPRDX5+siGPX4 is administered to the CRPC mice to observe the PRDX5 enzyme activity in the mice and to determine the therapeutic effect of the drug on a prostate cancer. The results show that siPRDX5 or siGPX4 or siPRDX5+siGPX4 can effectively inhibit the progression of the prostate cancer in CRPC mice by inhibiting the PRDX5 enzyme activity.

The first object of the present disclosure is to provide a double-stranded siRNA molecule as shown in any one of the following (A) to (C):
(A) siPRDX5, an siRNA molecule that inhibits the expression of the PRDX5 gene, having a double-stranded siRNA molecule formed by an RNA single strand set forth in SEQ ID NO. 1 and an RNA single strand set forth in SEQ ID NO. 2 which are complementary to each other;
(B) siGPX4, an siRNA molecule that inhibits the expression of a GPX4 gene, having a double-stranded siRNA molecule formed by an RNA single strand set forth in SEQ ID NO. 3 and an RNA single strand set forth in SEQ ID NO. 4 which are complementary to each other, or a double-stranded siRNA molecule formed by an RNA single strand set forth in SEQ ID NO. 5 and an RNA single strand set forth in SEQ ID NO. 6 which are complementary to each other; and
(C) a composition containing (A) and (B).

In one embodiment of the present disclosure, at least one nucleotide in the double-stranded siRNA molecule is a modified nucleotide.

In one embodiment of the present disclosure, all of the nucleotides in the double-stranded siRNA molecule are modified nucleotides.

In one embodiment of the present disclosure, the modification is selected from at least one of phosphorothioate, 2'-F, 2'-OMe, 2'-Ara-F, 2'-O-MO, m⁶A or m⁵C.

In one embodiment of the present disclosure, the modification is as shown in any one of the following (1) to (7):

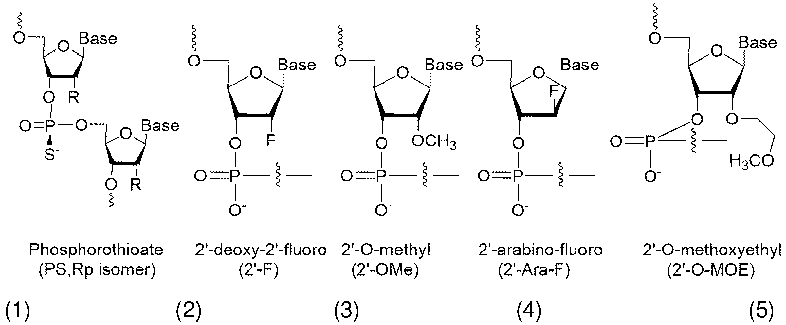

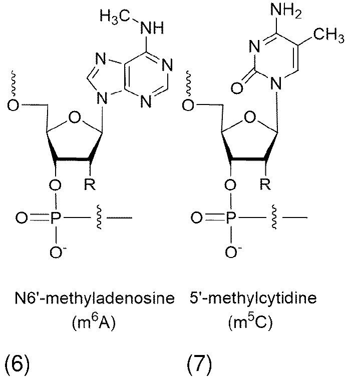

In one embodiment of the present disclosure, the double-stranded siRNA molecule is as shown in any one of the following (A1)-(A16), (B1)-(B16), and (C1)-(C16), where (A1)-(A16) are double-stranded siRNAs where the siPRDX5 is modified, and (B1)-(B16) and (C1)-(C16) are double-stranded siRNAs where the siGPX4 is modified:
(A1):
   5' (C)-[A]-(G)[A](C)[U](U)[A](UUA)[C](U)[A](G)[A](U)[G](A)[U](T) 3';
   5' [A]-(A)-[U](C)[A](U)[C](U)[A](G)[UAA](U)[A](A)[G](U)[C](U)[G]-[U]-[C] 3';
(A2)
   5' (C)-[A]-(G)[A](C)[U](U)[A](UUA)[C](U)[A](G)[A](U)[A](A)[U](T) 3';
   5' [A]-(A)-[U](C)[A](U)[C](U)[A](G)[UAA](U)[A](A)[G](U)[C](U)[G]-[U]-[C] 3';
(A3)
   5' (C)-[A]-(G)[A](C)[U](U)[A](UUA)[C](U)[A](G)[A](U)[G](A)[U](T) 3';
   5'[A]-(A)-[U](C)[A](U)[C](U)[A](G)[UAA](U)[A](A)[G](U)[C](U)[G]-[U]-[C] 3';
(A4)
   5' (C)-[A]-(G)[A](C)[U](U)[A](UUA)[C](U)[A](G)[A](U)[G](A)[U](T) 3';
   5'[A]-(A)-[U](C)[A](U)[C](U)[A](G)[UAA](U)[A](A)[G](U)[C](U)[G]-[U]-[C] 3';
(A5)
   5' (C)-[A*]-(G)[A*](C)[U](U)[A*](UUA*)[C](U)[A*](G)[A*](U)[G](A*)[U](T) 3';
   5' [A*]-(A*)-[U](C)[A*](U)[C](U)[A*](G)[UA*A*](U)[A*](A*)[G](U)[C](U)[G]-[U]-[C] 3';
(A6)
   5'(C')-[A]-(G)[A](C')[U](U)[A](UUA)[C'](U)[A](G)[A](U)[G](A)[U](T) 3';
   5' [A]-(A)-[U](C')[A](U)[C'](U)[A](G)[UAA](U)[A](A)[G](U)[C'](U)[G]-[U]-[C'] 3';
(A7)
   5' (C)-[A*]-(G)[A*](C)[U](U)[A*](UUA*)[C](U)[A*](G)[A*](U)[G](A*)[U](T) 3';
   5'[A*]-(A*)-[U](C)[A*](U)[C](U)[A*](G)[UA*A*](U)[A*](A*)[G](U)[C](U)[G]-[U]-[C] 3';
(A8)
   5' (C)-[A*]-(G)[A*](C)[U](U)[A*](UUA*)[C](U)[A*](G)[A*](U)[G](A*)[U](T) 3';
   5' [A*]-(A*)-[U](C)[A*](U)[C](U)[A*](G)[UA*A*](U)[A*](A*)[G](U)[C](U)[G]-[U]-[C] 3';
(A9)
   5' (C)-[A*]-(G)[A*](C)[U](U)[A*](UUA*)[C](U)[A*](G)[A*](U)[G](A*)[U](T) 3';
   5' [A*]-(A*)-[U](C)[A*](U)[C](U)[A*](G)[UA*A*](U)[A*](A*)[G](U)[C](U)[G]-[U]-[C] 3';
(A10)
   5' (C')-[A]-(G)[A](C')[U](U)[A](UUA)[C'](U)[A](G)[A](U)[G](A)[U](T) 3';
   5' [A]-(A)-[U](C')[A](U)[C'](U)[A'](G)[UAA](U)[A](A)[G](U)[C'](U)[G]-[U]-[C'] 3';
(A11)
   5'(C')-[A]-(G)[A](C')[U](U)[A](UUA)[C'](U)[A](G)[A](U)[G](A)[U](T) 3';
   5'[A]-(A)-[U](C')[A](U)[C'](U)[A](G)[UAA](U)[A](A)[G](U)[C'](U)[G]-[U]-[C'] 3';
(A12)
   5' (C')-[A]-(G)[A](C')[U](U)[A](UUA)[C'](U)[A](G)[A](U)[G](A)[U](T) 3';
   5' [A]-(A)-[U](C')[A](U)[C'](U)[A](G)[UAA](U)[A](A)[G](U)[C'](U)[G]-[U]-[C'] 3';
(A13)
   5' (C')-[A*]-(G)[A*](C')[U](U)[A*](UUA*)[C'](U)[A*](G)[A*](U)[G](A*)[U](T) 3';
   5'[A*]-(A*)-[U](C')[A*](U)[C'](U)[A*](G)[UA*A*](U)[A*](A*)[G](U)[C'](U)[G]-[U]-[C']3';
(A14)
   5' (C')-[A*]-(G)[A*](C')[U](U)[A*](UUA*)[C'](U)[A*](G)[A*](U)[G](A*)[U](T) 3';
   5'[A*]-(A*)-[U](C')[A*](U)[C'](U)[A*](G)[UA*A*](U)[A*](A*)[G](U)[C'](U)[G]-[U]-[C']3';
(A15)
   5' (C')-[A*]-(G)[A*](C')[U](U)[A*](UUA*)[C'](U)[A*](G)[A*](U)[G](A*)[U](T) 3';
   5'[A*]-(A*)-[U](C')[A*](U)[C'](U)[A*](G)[UA*A*](U)[A*](A*)[G](U)[C'](U)[G]-[U]-[C']3';
(A16)
   5' (C')-[A*]-(G)[A*](C')[U](U)[A*](UUA*)[C'](U)[A*](G)[A*](U)[G](A*)[U](T) 3';
   5'[A*]-(A*)-[U](C')[A*](U)[C'](U)[A*](G)[UA*A*](U)[A*](A*)[G](U)[C'](U)[G]-[U]-[C']3';
(B1)
   5' (A)-[C]-(A)[A](C)[G](U)[C](AAA)[U](U)[C](G)[A](U)[A](U)[G](U) 3';
   5' [A]-(C)-[A](U)[A](U)[C](G)[A](A)[UUU](G)[A](C)[G](U)[U](G)[U]-[A]-[C] 3';
(B2)
   5' (A)-[C]-(A)[A](C)[G](U)[C](AAA)[U](U)[C](G)[A](U)[A](U)[G](U) 3';
   5' [A]-(C)-[A](U)[A](U)[C](G)[A](A)[UUU](G)[A](C)[G](U)[U](G)[U]-[A]-[C] 3';
(B3)
   5' (A)-[C]-(A)[A](C)[G](U)[C](AAA)[U](U)[C](G)[A](U)[A](U)[G](U) 3';
   5' [A]-(C)-[A](U)[A](U)[C](G)[A](A)[UUU](G)[A](C)[G](U)[U](G)[U]-[A]-[C] 3';
(B4)
   5' (A)-[C]-(A)[A](C)[G](U)[C](AAA)[U](U)[C](G)[A](U)[A](U)[G](U) 3';
   5' [A]-(C)-[A](U)[A](U)[C](G)[A](A)[UUU](G)[A](C)[G](U)[U](G)[U]-[A]-[C] 3';
(B5)
   5' (A*)-[C]-(A*)[A*](C)[G](U)[C](A*A*A*)[U](U)[C](G)[A*](U)[A*](U)[G](U) 3';
   5' [A*]-(C)-[A*](U)[A*](U)[C](G)[A*](A*)[UUU](G)[A*](C)[G](U)[U](G)[U]-[A*]-[C] 3';
(B6)
   5' (A)-[C']-(A)[A](C')[G](U)[C'](AAA)[U](U)[C'](G)[A](U)[A](U)[G](U) 3';
   5' [A]-(C')-[A](U)[A](U)[C'](G)[A](A)[UUU](G)[A](C')[G](U)[U](G)[U]-[A]-[C'] 3';
(B7)
   5' (A*)-[C]-(A*)[A*](C)[G](U)[C](A*A*A*)[U](U)[C](G)[A*](U)[A*](U)[G](U) 3';
   5' [A*]-(C)-[A*](U)[A*](U)[C](G)[A*](A*)[UUU](G)[A*](C)[G](U)[U](G)[U]-[A*]-[C] 3';
(B8)
   5' (A*)-[C]-(A*)[A*](C)[G](U)[C](A*A*A*)[U](U)[C](G)[A*](U)[A*](U)[G](U) 3';
   5' [A*]-(C)-[A*](U)[A*](U)[C](G)[A*](A*)[UUU](G)[A*](C)[G](U)[U](G)[U]-[A*]-[C] 3';
(B9)
   5' (A*)-[C]-(A*)[A*](C)[G](U)[C](A*A*A*)[U](U)[C](G)[A*](U)[A*](U)[G](U) 3';
   5' [A*]-(C)-[A*](U)[A*](U)[C](G)[A*](A*)[UUU](G)[A*](C)[G](U)[U](G)[U]-[A*]-[C] 3';
(B10)
   5' (A)-[C']-(A)[A](C')[G](U)[C'](AAA)[U](U)[C'](G)[A](U)[A](U)[G](U) 3';
   5' [A]-(C')-[A](U)[A](U)[C'](G)[A](A)[UUU](G)[A](C')[G](U)[U](G)[U]-[A]-[C'] 3';
(B11)
   5' (A)-[C']-(A)[A](C')[G](U)[C'](AAA)[U](U)[C'](G)[A](U)[A](U)[G](U) 3';
   5' [A]-(C')-[A](U)[A](U)[C'](G)[A](A)[UUU](G)[A](C')[G](U)[U](G)[U]-[A]-[C'] 3';
(B12)
   5' (A)-[C']-(A)[A](C')[G](AAA)(U)[C'](G)[A](U)[A](U)[G](U) 3';
   5' [A]-(C')-[A](U)[A](U)[C'](G)[A](A)[UUU](G)[A](C')[G](U)[U](G)[U]-[A]-[C'] 3';
(B13)
   5' (A*)-[C']-(A*)[A*](C')[G](U)[C'](A*A*A*)[U](U)[C'](G)[A*](U)[A*](U)[G](U) 3';
   5' [A*]-(C')-[A*](U)[A*](U)[C'](G)[A*](A*)[UUU](G)[A*](C')[G](U)[U](G)[U]-[A*]-[C'] 3';
(B14)
   5' (A*)-[C']-(A*)[A*](C')[G](U)[C'](A*A*A*)[U](U)[C'](G)[A*](U)[A*](U)[G](U) 3';
   5' [A*]-(C')-[A*](U)[A*](U)[C'](G)[A*](A*)[UUU](G)[A*](C')[G](U)[U](G)[U]-[A*]-[C'] 3';
(B15)
   5' (A*)-[C']-(A*)[A*](C')[G](U)[C'](A*A*A*)[U](U)[C'](G)[A*](U)[A*](U)[G](U) 3';
   5' [A*]-(C')-[A*](U)[A*](U)[C'](G)[A*](A*)[UUU](G)[A*](C')[G](U)[U](G)[U]-[A*]-[C'] 3';
(B16)
   5' (A*)-[C']-(A*)[A*](C')[G](U)[C'](A*A*A*)[U](U)[C'](G)[A*](U)[A*](U)[G](U) 3';
   5' [A*]-(C')-[A*](U)[A*](U)[C'](G)[A*](A*)[UUU](G)[A*](C')[G](U)[U](G)[U]-[A*]-[C'] 3';
(C1)
   5' (G)-[U]-(G)[A](G)[G](C)[A](AGA)[C](C)[G](A)[A](G)[U](A)[A](A) 3';
   5' [U]-(U)-[U](A)[C](U)[U](C)[G](G)[UCU](U)[G](C)[C](U)[C](A)[C]-[U]-[G] 3';
(C2)
   5' (G)-[U]-(G)[A](G)[G](C)[A](AGA)[C](C)[G](A)[A](G)[U](A)[A](A) 3';
   5' [U]-(U)-[U](A)[C](U)[U](C)[G](G)[UCU](U)[G](C)[C](U)[C](A)[C]-[U]-[G] 3';
(C3)
   5' (G)-[U]-(G)[A](G)[G](C)[A](AGA)[C](C)[G](A)[A](G)[U](A)[A](A) 3';
   5' [U]-(U)-[U](A)[C](U)[U](C)[G](G)[UCU](U)[G](C)[C](U)[C](A)[C]-[U]-[G] 3';
(C4)
   5' (G)-[U]-(G)[A](G)[G](C)[A](AGA)[C](C)[G](A)[A](G)[U](A)[A](A) 3';
   5' [U]-(U)-[U](A)[C](U)[U](C)[G](G)[UCU](U)[G](C)[C](U)[C](A)[C]-[U]-[G] 3';
(C5)
   5' (G)-[U]-(G)[A*](G)[G](C)[A*](A*GA*)[C](C)[G](A*)[A*](G)[U](A*)[A*](A*) 3';
   5' [U]-(U)-[U](A*)[C](U)[U](C)[G](G)[UCU](U)[G](C)[C](U)[C](A*)[C]-[U]-[G] 3';
(C6)
   5'(G)-[U]-(G)[A](G)[G](C')[A](AGA)[C'](C')[G](A)[A](G)[U](A)[A](A) 3';
   5' [U]-(U)-[U](A)[C'](U)[U](C')[G](G)[UC'U](U)[G](C')[C'](U)[C'](A)[C']-[U]-[G] 3';
(C7)
   5' (G)-[U]-(G)[A*](G)[G](C)[A*](A*GA*)[C](C)[G](A*)[A*](G)[U](A*)[A*](A*) 3';
   5' [U]-(U)-[U](A*)[C](U)[U](C)[G](G)[UCU](U)[G](C)[C](U)[C](A*)[C]-[U]-[G] 3';
(C8)
   5' (G)-[U]-(G)[A*](G)[G](C)[A*](A*GA*)[C](C)[G](A*)[A*](G)[U](A*)[A*](A*) 3';
   5' [U]-(U)-[U](A*)[C](U)[U](C)[G](G)[UCU](U)[G](C)[C](U)[C](A*)[C]-[U]-[G] 3';
(C9)
   5' (G)-[U]-(G)[A*](G)[G](C)[A*](A*GA*)[C](C)[G](A*)[A*](G)[U](A*)[A*](A*) 3';
   5' [U]-(U)-[U](A*)[C](U)[U](C)[G](G)[UCU](U)[G](C)[C](U)[C](A*)[C]-[U]-[G] 3';
(C10)
   5' (G)-[U]-(G)[A](G)[G](C')[A](AGA)[C'](C')[G](A)[A](G)[U](A)[A](A) 3';
   5' [U]-(U)-[U](A)[C'](U)[U](C')[G](G)[UC'U](U)[G](C')[C'](U)[C'](A)[C']-[U]-[G] 3';
(C11)
   5' (G)-[U]-(G)[A](G)[G](C')[A](AGA)[C'](C')[G](A)[A](G)[U](A)[A](A) 3';
   5' [U]-(U)-[U](A)[C'](U)[U](C')[G](G)[UC'U](U)[G](C')[C'](U)[C'](A)[C']-[U]-[G] 3';
(C12)
   5' (G)-[U]-(G)[A](G)[G](C')[A](AGA)[C'](C')[G](A)[A](G)[U](A)[A](A) 3';
   5' [U]-(U)-[U](A)[C'](U)[U](C')[G](G)[UC'U](U)[G](C')[C'](U)[C'](A)[C']-[U]-[G] 3';
(C13)
   5' (G)-[U]-(G)[A*](G)[G](C')[A*](A*GA*)[C'](C')[G](A*)[A*](G)[U](A*)[A*](A*) 3';
   5' [U]-(U)-[U](A*)[C'](U)[U](C')[G](G)[UC'U](U)[G](C')[C'](U)[C'](A*)[C']-[U]-[G] 3';
(C14)
   5' (G)-[U]-(G)[A*](G)[G](C')[A*](A*GA*)[C'](C')[G](A*)[A*](G)[U](A*)[A*](A*) 3';
   5' [U]-(U)-[U](A*)[C'](U)[U](C')[G](G)[UC'U](U)[G](C')[C'](U)[C'](A*)[C']-[U]-[G] 3';
(C15)
   5' (G)-[U]-(G)[A*](G)[G](C')[A*](A*GA*)[C'](C')[G](A*)[A*](G)[U](A*)[A*](A*) 3';
   5' [U]-(U)-[U](A*)[C'](U)[U](C')[G](G)[UC'U](U)[G](C')[C'](U)[C'](A*)[C']-[U]-[G] 3';
(C16)
   5' (G)-[U]-(G)[A*](G)[G](C')[A*](A*GA*)[C'](C')[G](A*)[A*](G)[U](A*)[A*](A*) 3';
   5' [U]-(U)-[U](A*)[C'](U)[U](C')[G](G)[UC'U](U)[G](C')[C'](U)[C'](A*)[C']-[U]-[G] 3';
where A-, U-, C-, and G- denote phosphorothioate-modified ribonucleotides A, U, C, and G, respectively;
(A), (U), (C), and (G) denote 2'-F-modified ribonucleotides A, U, C, and G, respectively;
[A], [U], [C] and [G] denote 2'-OMe-modified ribonucleotides A, U, C and G, respectively;
(A), (U), (C) and (G) denote 2'-Ara-F-modified ribonucleotides A, U, C and G, respectively;
[A], [U], [C] and [G] denote 2'-O-MOE-modified ribonucleotides A, U, C and G, respectively;
A*, U*, C* and G* denote m⁶A-modified ribonucleotides A, U, C and G, respectively; and
A', U', C' and G' denote m⁵C-modified ribonucleotides A, U, C and G, respectively.

The second object of the present disclosure is to provide a biological material associated with the siRNA, which is any one of the following:
(1) a vector expressing the siRNA;
(2) a host cell carrying the siRNA or the vector;
(3) a reagent containing the siRNA, or the vector in (1) or the host cell in (2); and
(4) a pharmaceutical composition containing the siRNA.

In one embodiment of the present disclosure, the pharmaceutical composition may further contain an androgen or an androgen receptor inhibitor.

In one embodiment of the present disclosure, the androgen receptor inhibitor is selected from Enzalutamide (ENZ).

In one embodiment of the present disclosure, the pharmaceutical composition contains a pharmaceutically acceptable carrier or excipient.

In one embodiment of the present disclosure, the pharmaceutically acceptable carrier comprises a microliposome, a microcell, a metal particle, or a polymer particle.

The third object of the present disclosure is to provide application of the siRNA or the biological material in preparation of drugs for the prevention or treatment of a prostate cancer.

In one embodiment of the present disclosure, the prostate cancer is an advanced prostate cancer or castration-resistant prostate cancer.

The fourth object of the present disclosure is to provide a method for preventing or treating a prostate cancer comprising administrating the siRNA or the biological material to a subject.

The fifth object of the present disclosure is to provide a method for inhibiting the expression of the PRDX5 and/or GPX4 genes in a cell, where the method comprises contacting an effective amount of the siRNA or the biological material with a cell, thereby inhibiting the expression of the PRDX5 and/or GPX4 genes in the cell.

### Beneficial effects:

1. The present disclosure proposes for the first time that interfering with or silencing the expression of the PRDX5 and/or GPX4 genes can ameliorate the prostate cancer, especially the castration-resistant prostate cancer, has a significant killing effect on the castration-resistant prostate cancer cells with a cell inhibition rate of up to 50% around; reduces the prostate weight in drug-resistant CRPC mice, inhibits the growth of tumors, reduces the prostate weight to about 40 mg by combining with the androgen receptor inhibitor ENZ; and significantly inhibits PRDX5 enzyme activity in the prostate.
2. The present disclosure proposes for the first time that the preparation of a drug for treating CRPC utilizing the nucleic acid drugs siPRDX5 and/or siPGX4 will promote the application of the nucleic acid drugs in the clinical treatment of the prostate cancer and is of great significance. Drug research takes an average of 8-10 years from a compound molecule to the clinic, and requires a lot of human and material support, with huge time and economic costs. The scheme of the present disclosure can greatly shorten the time from drug discovery to clinical translation.

### Brief Description of the Drawings

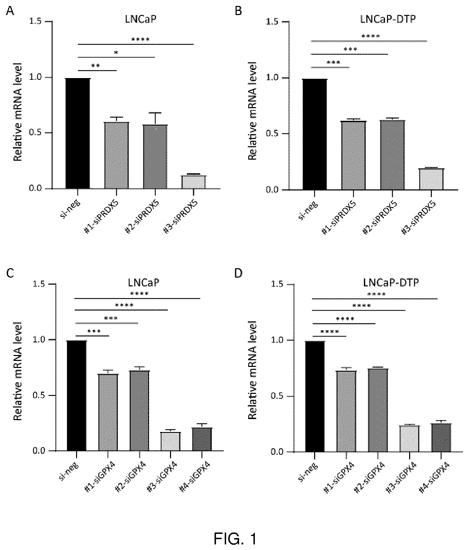

FIG. 1 shows the mRNA expression levels of PRDX5 and GPX4 in LNCaP and LNCaP-derived DTP cells (LNCaP-DTP) determined by qRT-PCR after the addition of siRNAs of #1-siPRDX5, #2-siPRDX5, and #3-siPRDX5 as well as #1-siGPX4, #2-siGPX4, #3-siGPX4, and #4-siGPX4 provided by the present disclosure, respectively, in which FIG. A shows the relative expression level of PRDX5 in LNCaP cells; FIG. B shows the relative expression level of PRDX5 in LNCaP-DTP cells; FIG. C shows the relative expression level of GPX4 in LNCaP cells; and FIG. D shows the relative expression level of GPX4 in LNCaP-DTP cells.

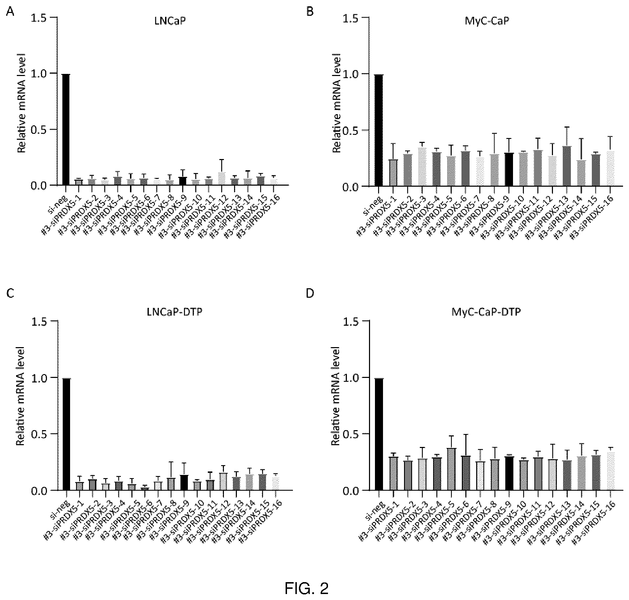

FIG. 2 shows the mRNA expression levels of PRDX5 in LNCaP, MyC-CaP, LNCaP-derived DTP cells (LNCaP-DTP), and MyC-CaP-derived DTP cells (MyC-CaP-DTP) determined by qRT-PCR after the addition of modified #3-siPRDX5 provided by the present disclosure, in which FIG. A shows the LNCaP cells; FIG. B shows MyC-CaP cells; FIG. C shows LNCaP-DTP cells; and FIG. D shows MyC-CaP-DTP cells.

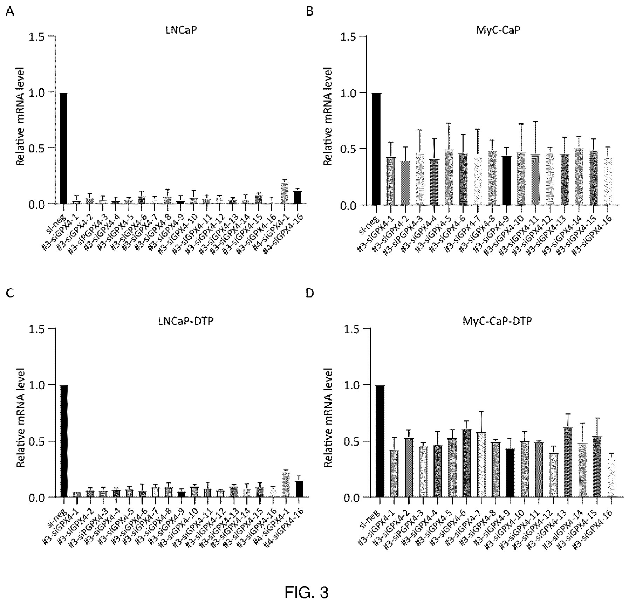

FIG. 3 shows the mRNA expression levels of GPX4 in LNCaP, MyC-CaP, LNCaP-derived DTP cells (LNCaP-DTP), and MyC-CaP-derived DTP cells (MyC-CaP-DTP) determined by qRT-PCR after the addition of modified #3-siGPX4 and #4-siGPX4 provided in the present disclosure, in which FIG. A shows LNCaP cells; FIG. B shows MyC-CaP cells; FIG. C shows LNCaP-DTP cells; and FIG. D shows MyC-CaP-DTP cells.

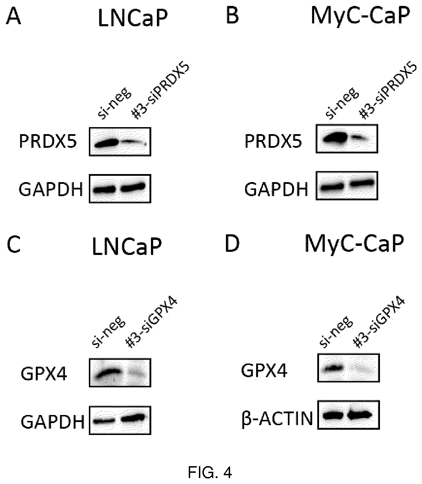

FIG. 4 shows the changes in the expression of PRDX5 or GPX4 proteins in LNCaP and MyC-CaP cells determined by WB after the addition of unmodified #3-siPRDX5 or #3-siGPX4, in which FIG. A shows the PRDX5 protein expression as determined in LNCaP cells; FIG. B shows the PRDX5 protein expression as determined in MyC-CaP cells; FIG. C shows the GPX4 protein expression as determined in LNCaP cells; and FIG. D shows the GPX4 protein expression as determined in MyC-CaP cells.

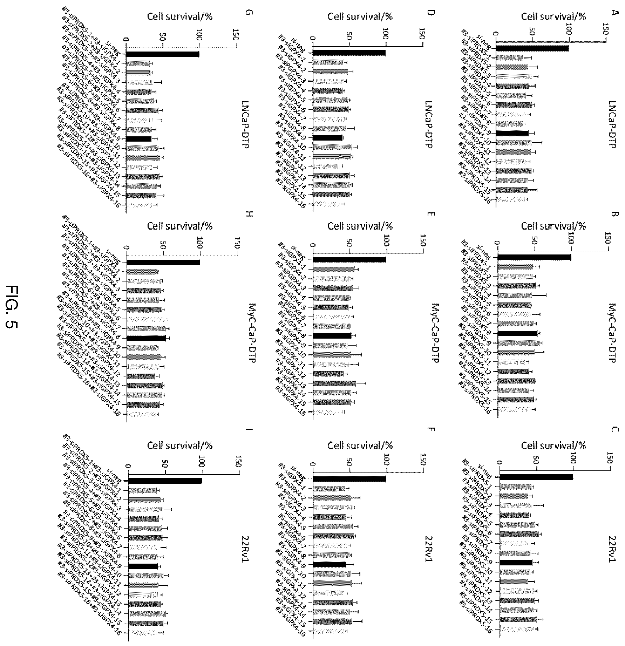

FIG. 5 shows the cell survival in LNCaP-DTP and MyC-CaP-DTP cells determined by CCK8 after the addition of modified #3-siPRDX5-1-16 or modified #3-siGPX4-1-16 or modified #3-siPRDX5-1-16 + modified #3-siGPX4-1-16, in which FIG. A shows the effect of the addition of siPRDX5-1-16 on the survival of LNCaP-DTP cells; FIG. B shows the effect of the addition of siPRDX5-1-16 on the survival of MyC-CaP-DTP cells; FIG. C shows the effect of the addition of siPRDX5-1-16 on the survival of 22Rv1 cells; FIG. D shows the effect of the addition of siGPX4-1-16 on the survival of LNCaP-DTP cells; FIG. E shows the effect of the addition of siGPX4-1-16 on the survival of MyC-CaP-DTP cells; FIG. F shows the effect of the addition of siGPX4-1-16 on the survival of 22Rv1 cells; FIG. G shows the effect of the addition of siPRDX5-1-16 + siGPX4-1-16 on the survival of LNCaP-DTP cells; FIG. H shows the effect of the addition of siPRDX5-1-16 + siGPX4-1-16 on the survival of MyC-CaP-DTP cells; and FIG. I shows the effect of the addition of siPRDX5-1-16 + siGPX4-1-16 on the survival of 22Rv1 cells.

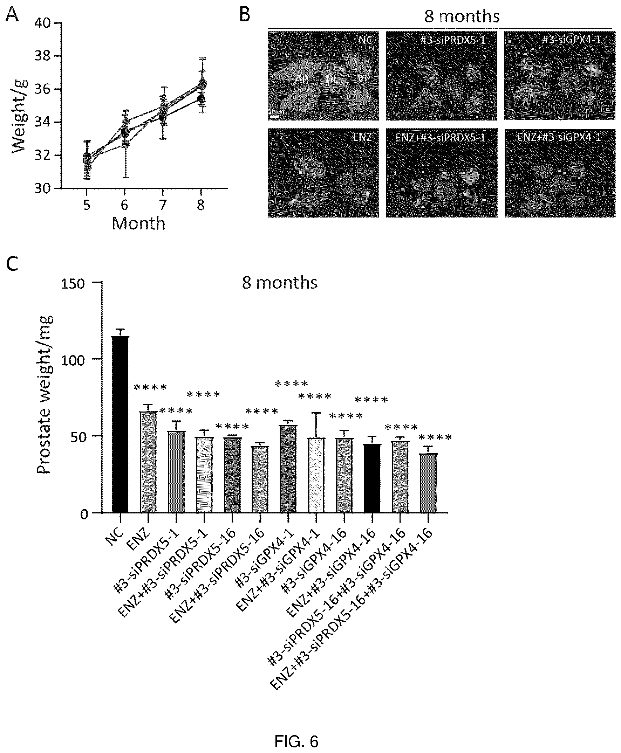

FIG. 6 is a graph showing the effect of NC control, ENZ, #3-siPRDX5-1, #3-siPRDX5-16, #3-siGPX4-1, #3-siGPX4-16, #3-siPRDX5-16 + #3-siGPX4-16, and their co-administration with ENZ on the change in prostate weight for CRPC formed by continuous administration of ENZ in C-*MYC*-overexpressing prostate cancer mice, in which FIG. A is a graph showing the change in body weight of mice in each group (NC, ENZ, siPRDX5-1, and ENZ + siPRDX5-1 groups) vs administration; FIG. B is a comparative graph showing the photographs of the prostate removed from 8-month-old mice in each group; and FIG. C is a graph showing the change in prostate weight of 8-month-old mice in each group.

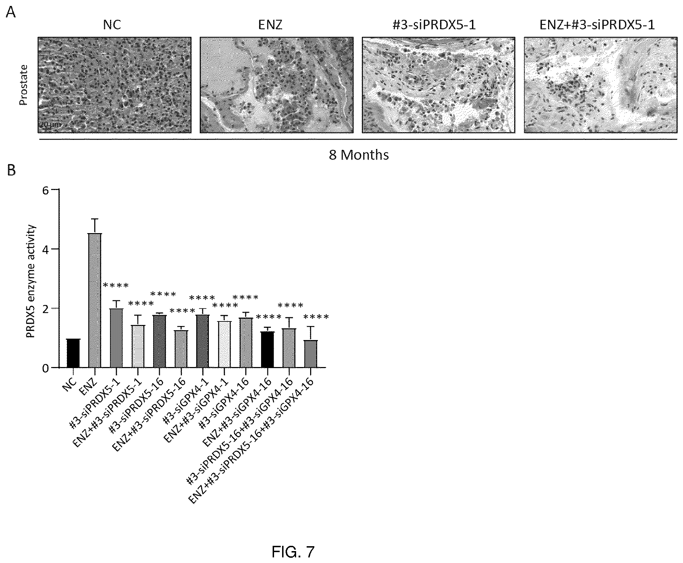

FIG. 7 is a graph showing the effect of NC control, ENZ, #3-siPRDX5-1, #3-siPRDX5-16, #3-siGPX4-1, #3-siGPX4-16, #3-siPRDX5-16 + #3-siGPX4-16, and their co-administration with ENZ on the change in PRDX5 enzyme activity for CRPC formed by continuous administration of ENZ in *C-MYC*-overexpressing prostate cancer mice, in which FIG. A is a graph showing the HE staining of prostate tissue sections from 8-month-old mice in each group; and FIG. B is a graph showing the quantitation of PRDX5 enzyme activity of prostate tissues from mice in each group.

### Detailed Description of Embodiments

The present disclosure is further described below in connection with the accompanying drawings of the specification and specific examples, and the examples do not limit the present disclosure in any way. Unless otherwise indicated, the reagents, methods and equipment employed in the present disclosure are those conventional in the art.

Unless specifically stated, the reagents and materials used in the following examples are commercially available.

### Example 1 Synthesis of unmodified siRNAs

siRNAs were designed based on the full mRNA sequences of human-derived PRDX5 and GPX4. siRNAs were preliminarily designed and synthesized by evaluating the activities of all candidate siRNAs based on the basic design principles of siRNAs. All the sequences were obtained from NCBI gene database.
(1) The sequence of siPRDX5 is:
   #1:
      5'-AGACAGACUUAUUACUAGAUGAUTC-3' (SEQ ID NO.7);
      5'-GAAUCAUCUAGUAAUAAGUCUGUCUCC-3' (SEQ ID NO.8);
   #2:
      5' GGCCAGAUUUCUGCAAUAAACACTT-3' (SEQ ID NO.9);
      5' AAGUGUUUAUUGCAGAAAUCUGGCCAA-3' (SEQ ID NO.10); and
   #3:
      5'-CAGACUUAUUACUAGAUGAUT-3' (SEQ ID NO.1);
      5'-AAUCAUCUAGUAAUAAGUCUGUC-3' (SEQ ID NO.2).
(2) The sequence of siGPX4 is:
   #1:
      5'-GUGAGGCAAGACCGAAGUAAACUAC- 3' (SEQ ID NO.11);
      5'-GUAGUUUACUUCGGUCUUGCCUCACUG-3' (SEQ ID NO.12);
   #2:
      5'-CUACAACGUCAAAUUCGAUAUGUTC-3' (SEQ ID NO.13);
      5'-GAACAUAUCGAAUUUGACGUUGUAGCC-3' (SEQ ID NO.14);
   #3:
      5'-ACAACGUCAAAUUCGAUAUGU-3' (SEQ ID NO.3);
      5'-ACAUAUCGAAUUUGACGUUGUAC-3' (SEQ ID NO.4); and
   #4:
      5'-GUGAGGCAAGACCGAAGUAAA-3' (SEQ ID NO.5);
      5'-UUUACUUCGGUCUUGCCUCACUG-3' (SEQ ID NO.6).

### Example 2 Gene interference ability of unmodified siPRDX5 and siGPX4 against prostate cancer cells and DTP cells

To screen for suitable siRNAs, the interference activity of the siRNAs designed in Example 1 was determined.

S1. LNCaP and MyC-CaP cells were seeded at 1 × 10⁶ in a 10 cm cell culture dish. On the second day, the cells were attached and then treated with 50 µM enzalutamide (ENZ) for 9 days, during which the medium was replaced with a fresh drug-containing medium (1640 medium containing 50 µM ENZ) for culture every three days. The cells were collected. After the treatment for 9 days, the cells were drug-resistant (DTP cells) and were labeled as LNCaP-DTP cells and MyC-CaP-DTP cells, respectively.

S2. LNCaP, MyC-CaP, and LNCaP-DTP and MyC-CaP-DTP cells as collected in S1, were seeded in a 6 cm dish and a 10 cm dish that contain fresh complete medium (1640 medium containing 10% fetal bovine serum and 1% secondary antibody), attached, and then transfected with siPRDX5 and siGPX4 designed in Example 1, and then mRNA and protein were extracted.

S3. RNA level and protein level were determined by qRT-PCR and Western Blot assays, and siRNAs with interference activity were screened and obtained.

The results are shown in FIG. 1 and FIG. 4 as well as Tables 1 and 2. The addition of siRNA of #3-siPRDX5 succeeded in substantially reducing the expression of PRDX5, including mRNA level and protein level; the addition of siRNAs of #3-siGPX4 and #4-siRNA succeeded in substantially reducing the expression of GPX4, including mRNA level and protein level. For human prostate cancer cells LNCaP and drug-resistant LNCaP-DTP cells, the mRNA expression level of PRDX5 interfered with siRNA of #3-siPRDX5 was reduced to around 0.16, whereas the mRNA expression level of PRDX5 interfered with siRNAs of #1-siPRDX5 and #2-siPRDX5 was only reduced to around 0.60. For human prostate cancer cells LNCaP and drug-resistant LNCaP-DTP cells, the mRNA expression level of GPX4 interfered with siRNA of #3-siGPX4 was reduced to around 0.21, and the mRNA expression level of GPX4 interfered with siRNA of #4-siGPX4 was reduced to around 0.24, whereas the mRNA expression level of GPX4 interfered with siRNAs of #1-siGPX4 and #2-siGPX4 was reduced to around 0.73.

It can be seen that the siRNAs as designed in Example 1 of the present disclosure all have the ability to interfere with the expression of the target gene to a certain extent, but among them, the ability of the siRNAs, #3-siPRDX5, #3-siGPX4 and #4-siGPX4, to interfere with the expression of the gene is superior.

### Example 3 Modification of siRNA and determination of interference ability

The siRNAs, #3-siPRDX5, #3-siGPX4 and #4-siGPX4, as screened in Example 2 were base-modified and synthesized at biotechnology company, where:
A-, U-, C-, and G- denote phosphorothioate-modified ribonucleotides A, U, C, and G, respectively;
(A), (U), (C), and (G) denote 2'-F-modified ribonucleotides A, U, C, and G, respectively;
[A], [U], [C] and [G] denote 2'-OMe-modified ribonucleotides A, U, C and G, respectively;
(A), (U), (C) and (G) denote 2'-Ara-F-modified ribonucleotides A, U, C and G, respectively;
[A], [U], [C] and [G] denote 2'-O-MOE-modified ribonucleotides A, U, C and G, respectively;
A*, U*, C* and G* denote m⁶A-modified ribonucleotides A, U, C and G, respectively; and
A', U', C' and G' denote m⁵C-modified ribonucleotides A, U, C and G, respectively.

The base-modified sequences for siRNA (#3-siPRDX5) are shown in the following siPRDX5-1 to siPRDX5-16:
siPRDX5-1
   5' (C)-[A]-(G)[A](C)[U](U)[A](UUA)[C](U)[A](G)[A](U)[G](A)[U](T) 3';
   5' [A]-(A)-[U](C)[A](U)[C](U)[A](G)[UAA](U)[A](A)[G](U)[C](U)[G]-[U]-[C] 3';
siPRDX5-2
   5' (C)-[A]-(G)[A](C)[U](U)[A](UUA)[C](U)[A](G)[A](U)[G](A)[U](T) 3';
   5' [A]-(A)-[U](C)[A](U)[C](U)[A](G)[UAA](U)[A](A)[G](U)[C](U)[G]-[U]-[C] 3';
siPRDX5-3
   5' (C)-[A]-(G)[A](C)[U](U)[A](UUA)[C](U)[A](G)[A](U)[G](A)[U](T) 3';
   5' [A]-(A)-[U](C)[A](U)[C](U)[A](G)[UAA](U)[A](A)[G](U)[C](U)[G]-[U]-[C] 3';
siPRDX5-4
   5' (C)-[A]-(G)[A](C)[U](U)[A](UUA)[C](U)[A](G)[A](U)[G](A)[U](T) 3';
   5' [A]-(A)-[U](C)[A](U)[C](U)[A](G)[UAA](U)[A](A)[G](U)[C](U)[G]-[U]-[C] 3';
siPRDX5-5
   5' (C)-[A*]-(G)[A*](C)[U](U)[A*](UUA*)[C](U)[A*](G)[A*](U)[G](A*)[U](T) 3';
   5' [A*]-(A*)-[U](C)[A*](U)[C](U)[A*](G)[UA*A*](U)[A*](A*)[G](U)[C](U)[G]-[U]-[C] 3';
siPRDX5-6
   5' (C')-[A]-(G)[A](C')[U](U)[A](UUA)[C'](U)[A](G)[A](U)[G](A)[U](T) 3';
   5' [A]-(A)-[U](C')[A](U)[C'](U)[A](G)[UAA](U)[A](A)[G](U)[C'](U)[G]-[U]-[C'] 3';
siPRDX5-7
   5' (C)-[A*]-(G)[A*](C)[U](U)[A*](UUA*)[C](U)[A*](G)[A*](U)[G](A*)[U](T) 3';
   5'[A*]-(A*)-[U](C)[A*](U)[C](U)[A*](G)[UA*A*](U)[A*](A*)[G](U)[C](U)[G]-[U]-[C] 3';
siPRDX5-8
   5' (C)-[A*]-(G)[A*](C)[U](U)[A*](UUA*)[C](U)[A*](G)[A*](U)[G](A*)[U](T) 3';
   5' [A*]-(A*)-[U](C)[A*](U)[C](U)[A*](G)[UA*A*](U)[A*](A*)[G](U)[C](U)[G]-[U]-[C] 3';
siPRDX5-9
   5' (C)-[A*]-(G)[A*](C)[U](U)[A*](UUA*)[C](U)[A*](G)[A*](U)[G](A*)[U](T) 3';
   5' [A*]-(A*)-[U](C)[A*](U)[C](U)[A*](G)[UA*A*](U)[A*](A*)[G](U)[C](U)[G]-[U]-[C] 3';
siPRDX5-10
   5' (C')-[A]-(G)[A](C')[U](U)[A](UUA)[C'](U)[A](G)[A](U)[G](A)[U](T) 3';
   5' [A]-(A)-[U](C')[A](U)[C'](U)[A](G)[UAA](U)[A](A)[G](U)[C'](U)[G]-[U]-[C'] 3';
siPRDX5-11
   5' (C')-[A]-(G)[A](C')[U](U)[A](UUA)[C'](U)[A](G)[A](U)[G](A)[U](T) 3';
   5' [A]-(A)-[U](C')[A](U)[C'](U)[A](G)[UAA](U)[A](A)[G](U)[C'](U)[G]-[U]-[C'] 3';
siPRDX5-12
   5' (C')-[A]-(G)[A](C')[U](U)[A](UUA)[C'](U)[A](G)[A](U)[G](A)[U](T) 3';
   5' [A]-(A)-[U](C')[A](U)[C'](U)[A](G)[UAA](U)[A](A)[G](U)[C'](U)[G]-[U]-[C'] 3';
siPRDX5-13
   5' (C')-[A*]-(G)[A*](C')[U](U)[A*](UUA*)[C'](U)[A*](G)[A*](U)[G](A*)[U](T) 3';
   5'[A*]-(A*)-[U](C')[A*](U)[C'](U)[A*](G)[UA*A*](U)[A*](A*)[G](U)[C'](U)[G]-[U]-[C']3';
siPRDX5-14
   5' (C')-[A*]-(G)[A*](C')[U] (U)[A*](UUA*)[C'](U)[A*](G)[A*](U)[G](A*)[U](T) 3';
   5'[A*]-(A*)-[U](C')[A*](U[C'](U)[A*](G)[UA*A*](U)[A*](A*)[G](U)[C'](U)[G]-[U]-[C']3';
siPRDX5-15
   5' (C')-[A*]-(G)[A*](C')[U](U)[A*](UUA*)[C'](U)[A*](G)[A*](U)[G](A*)[U](T) 3';
   5'[A*]-(A*)-[U](C')[A*](U)[C'](U)[A*](G)[UA*A*](U)[A*](A*)[G](U)[C'](U)[G]-[U]-[C']3';
siPRDX5-16
   5' (C')-[A*]-(G)[A*](C')[U](U)[A*](UUA*)[C'](U)[A*](G)[A*](U)[G](A*)[U](T) 3';
   5'[A*]-(A*)-[U](G')[A*](U)[G'](U)[A*](G)[UA*A*](U)[A*](A*)[G](U)[C'](U)[G]-[U]-[C']3'.

The base-modified sequences for siRNA (#3-siGPX4) are shown in the following siGPX4-1 to siGPX4-16:
siGPX4-1
   5' (A)-[C]-(A)[A](C)[G](U)[C](AAA)[U](U)[C](G)[A](U)[A](U)[G](U) 3';
   5' [A]-(C)-[A](U)[A](U)[C](G)[A](A)[UUU](G)[A](C)[G](U)[U](G)[U]-[A]-[C] 3';
siGPX4-2
   5' (A)-[C]-(A)[A](C)[G](U)[C](AAA)[U](U)[C](G)[A](U)[A](U)[G](U) 3';
   5' [A]-(C)-[A](U)[A](U)[C](G)[A](A)[UUU](G)[A](C)[G](U)[U](G)[U]-[A]-[C] 3';
siGPX4-3
   5' (A)-[C]-(A)[A](C)[G](U)[C](AAA)[U](U)[C](G)[A](U)[A](U)[G](U) 3';
   5' [A]-(C)-[A](U)[A](U)[C](G)[A](A)[UUU](G)[A](C)[G](U)[U](G)[U]-[A]-[C] 3';
siGPX4-4
   5' (A)-[C]-(A)[A](C)[G](U)[C](AAA)[U](U)[C](G)[A](U)[A](U)[G](U) 3';
   5' [A]-(C)-[A](U)[A](U)[C](G)[A](A)[UUU](G)[A](C)[G](U)[U](G)[U]-[A]-[C] 3';
siGPX4-5
   5' (A*)-[C]-(A*)[A*](C)[G](U)[C](A*A*A*)[U](U)[C](G)[A*](U)[A*](U)[G](U) 3';
   5' [A*]-(C)-[A*](U)[A*](U)[C](G)[A*](A*)[UUU](G)[A*](C)[G](U)[U](G)[U]-[A*]-[C] 3';
siGPX4-6
   5' (A)-[C']-(A)[A](C')[G](U)[C'](AAA)[U](U)[C'](G)[A](U)[A](U)[G](U) 3';
   5' [A]-(C')-[A](U)[A](U)[C'](G)[A](A)[UUU](G)[A](C')[G](U)[U](G)[U]-[A]-[C'] 3';
siGPX4-7
   5' (A*)-[C]-(A*)[A*](C)[G](U)[C](A*A*A*)[U](U)[C](G)[A*](U)[A*](U)[G](U) 3';
   5' [A*]-(C)-[A*](U)[A*](U)[C](G)[A*](A*)[UUU](G)[A*](C)[G](U)[U](G)[U]-[A*]-[C] 3';
siGPX4-8
   5' (A*)-[C]-(A*)[A*](C)[G](U)[C](A*A*A*)[U](U)[C](G)[A*](U)[A*](U)[G](U) 3';
   5' [A*]-(C)-[A*](U)[A*](U)[C](G)[A*](A*)[UUU](G)[A*](C)[G](U)[U](G)[U]-[A*]-[C] 3';
siGPX4-9
   5' (A*)-[C]-(A*)[A*](C)[G](U)[C](A*A*A*)[U](U)[C](G)[A*](U)[A*](U)[G](U) 3';
   5' [A*]-(C)-[A*](U)[A*](U)[C](G)[A*](A*)[UUU](G)[A*](C)[G](U)[U](G)[U]-[A*]-[C] 3';
siGPX4-10
   5' (A)-[C']-(A)[A](C')[G](U)[C'](AAA)[U](U)[C'](G)[A](U)[A](U)[G](U) 3';
   5' [A]-(C')-[A](U)[A](U)[C'](G)[A](A)[UUU](G)[A](C')[G](U)[U](G)[U]-[A]-[C'] 3';
siGPX4-11
   5' (A)-[C']-(A)[A](C')[G](U)[C'](AAA)[U](U)[C'](G)[A](U)[A](U)[G](U) 3';
   5' [A]-(C')-[A](U)[A](U)[C'](G)[A](A)[UUU](G)[A](C')[G](U)[U](G)[U]-[A]-[C'] 3';
siGPX4-12
   5' (A)-[C']-(A)[A](C')[G](U)[C'](AAA)[U] (U)[C'](G)[A](U)[A](U)[G](U) 3';
   5' [A]-(C')-[A](U)[A](U)[C'](G)[A](A)[UUU](G)[A](C')[G](U)[U](G)[U]-[A]-[C'] 3';
siGPX4-13
   5' (A*)-[C']-(A*)[A*](C')[G](U)[C'](A*A*A*)[U](U)[C'](G)[A*](U)[A*](U)[G](U) 3';
   5' [A*]-(C')-[A*](U)[A*](U)[C'](G)[A*](A*)[UUU](G)[A*](C')[G](U)[U](G)[U]-[A*]-[C'] 3';
siGPX4-14
   5' (A*)-[C']-(A*)[A*](C')[G](U)[C'](A*A*A*)[U](U)[C'](G)[A*](U)[A*](U)[G](U) 3';
   5' [A]-(C')-[A*](U)[A*](U)[C'](G)[A*](A*)[UUU](G)[A*](C')[G](U)[U](G)[U]-[A*]-[C'] 3';
siGPX4-15
   5' (A*)-[C']-(A*)[A*](C')[G](U)[C'](A*A*A*)[U](U)[C'](G)[A*](U)[A*](U)[G](U) 3';
   5' [A*]-(C')-[A*](U)[A*](U)[C'](G)[A*](A*)[UUU](G)[A*](C')[G](U)[U](G)[U]-[A*]-[C'] 3';
siGPX4-16
   5' (A*)-[C']-(A*)[A*](C')[G](U)[C'](A*A*A*)[U](U)[C'](G)[A*](U)[A*](U)[G](U) 3';
   5' [A*]-(C')-[A*](U)[A*](U)[C'](G)[A*](A*)[UUU](G)[A*](C')[G](U)[U](G)[U]-[A*]-[C'] 3';

The base-modified sequences for siRNA (#4-siGPX4), are shown in the following siGPX4-(1) to siGPX4-(16):
siGPX4-(1)
   5' (G)-[U]-(G)[A](G)[G](C)[A](AGA)[C](C)[G](A)[A](G)[U](A)[A](A) 3';
   5' [U]-(U)-[U](A)[C](U)[U](C)[G](G)[UCU](U)[G](C)[C](U)[C](A)[C]-[U]-[G] 3';
siGPX4-(2)
   5' (G)-[U]-(G)[A](G)[G](C)[A](AGA)[C](C)[G](A)[A](G)[U](A)[A](A) 3';
   5' [U]-(U)-[U](A)[C](U)[U](C)[G](G)[UCU](U)[G](C)[C](U)[C](A)[C]-[U]-[G] 3';
siGPX4-(3)
   5' (G)-[U]-(G)[A](G)[G](C)[A](AGA)[C](C)[G](A)[A](G)[U](A)[A](A) 3';
   5' [U]-(U)-[U](A)[C](U)[U](C)[G](G)[UCU](U)[G](C)[C](U)[C](A)[C]-[U]-[G] 3';
siGPX4-(4)
   5' (G)-[U]-(G)[A](G)[G](C)[A](AGA)[C](C)[G](A)[A](G)[U](A)[A](A) 3';
   5' [U]-(U)-[U](A)[C](U)[U](C)[G](G)[UCU](U)[G](C)[C](U)[C](A)[C]-[U]-[G] 3';
siGPX4-(5)
   5' (G)-[U]-(G)[A*](G)[G](C)[A*](A*GA*)[C](C)[G](A*)[A*](G)[U](A*)[A*](A*) 3';
   5' [U]-(U)-[U](A*)[C](U)[U](C)[G](G)[UCU](U)[G](C)[C](U)[C](A*)[C]-[U]-[G] 3';
siGPX4-(6)
   5' (G)-[U]-(G)[A](G)[G](C')[A](AGA)[C'](C')[G](A)[A](G)[U](A)[A](A) 3';
   5' [U]-(U)-[U](A)[C'](U)[U](C')[G](G)[UC'U](U)[G](C')[C'](U)[C'](A)[C']-[U]-[G] 3';
siGPX4-(7)
   5' (G)-[U]-(G)[A*](G)[G](C)[A*](A*GA*)[C](C)[G](A*)[A*](G)[U](A*)[A*](A*) 3';
   5' [U]-(U)-[U](A*)[C](U)[U](C)[G](G)[UGU](U)[G](C)[C](U)[C](A*)[C]-[U]-[G] 3';
siGPX4-(8)
   5' (G)-[U]-(G)[A*](G)[G](C)[A*](A*GA*)[C](C)[G](A*)[A*](G)[U](A*)[A*](A*) 3';
   5' [U]-(U)-[U](A*)[C](U)[U](C)[G](G)[UCU](U)[G](C)[C](U)[C](A*)[C]-[U]-[G] 3';
siGPX4-(9)
   5' (G)-[U]-(G)[A*](G)[G](C)[A*](A*GA*)[C](C)[G](A*)[A*](G)[U](A*)[A*](A*) 3';
   5' [U]-(U)-[U](A*)[C](U)[U](C)[G](G)[UCU](U)[G](C)[C](U)[C](A*)[C]-[U]-[G] 3';
siGPX4-(10)
   5' (G)-[U]-(G)[A](G)[G](C')[A](AGA)[C'](C')[G](A)[A](G)[U](A)[A](A) 3';
   5' [U]-(U)-[U](A)[C'](U)[U](C')[G](G)[UC'U](U)[G](C')[C'](U)[C'](A)[C']-[U]-[G] 3';
siGPX4-(11)
   5' (G)-[U]-(G)[A](G)[G](C')[A](AGA)[C'](C')[G](A)[A](G)[U](A)[A](A) 3';
   5' [U]-(U)-[U](A)[C'](U)[U](C')[G](G)[UC'U](U)[G](C')[C'](U)[C'](A)[C']-[U]-[G] 3';
siGPX4-(12)
   5' (G)-[U]-(G)[A](G)[G](G')[C](AGA)[C'](C')[G](A)[A](G)[U](A)[A](A) 3';
   5' [U]-(U)-[U](A)[C'](U)[U](C')[G](G)[UC'U](U)[G](C')[C'](U)[C'](A)[C']-[U]-[G] 3';
siGPX4-(13)
   5' (G)-[U]-(G)[A*](G)[G](C')[A*](A*GA*)[C'](C')[G](A*)[A*](G)[U](A*)[A*](A*) 3';
   5' [U]-(U)-[U](A*)[C'](U)[U](C')[G](G)[UC'U](U)[G](C')[C'](U)[C'](A*)[C']-[U]-[G] 3';
siGPX4-(14)
   5' (G)-[U]-(G)[A*](G)[G](C')[A*](A*GA*)[C'](C')[G](A*)[A*](G)[U](A*)[A*](A*) 3';
   5' [U]-(U)-[U](A*)[C'](U)[U](C')[G](G)[UG'U](U)[G](C')[C'](U)[C'](A*)[C']-[U]-[G] 3';
siGPX4-(15)
   5' (G)-[U]-(G)[A*](G)[G](C')[A*](A*GA*)[C'](C')[G](A*)[A*](G)[U](A*)[A*](A*) 3';
   5' [U]-(U)-[U](A*)[C'](U)[U](C')[G](G)[UC'U](U)[G](C')[C'](U)[C'](A*)[C']-[U]-[G] 3';
siGPX4-(16)
   5' (G)-[U]-(G) [A*](G)[G](C')[A*](A*GA*)[C'](C')[G](A*)[A*](G)[U](A*)[A*](A*) 3';
   5' [U]-(U)-[U](A*)[C'](U)[U](C')[G](G)[UCU'](U)[G](C')[C'](U)[C'](A*)[C']-[U]-[G] 3'.

The results are shown in FIG. 2 and FIG. 3 as well as Tables 1 and 2. Using the same method as in Example 2, LNCaP and MyC-CaP cells as well as their derived LNCaP-DTP and MyC-CaP-DTP cells were treated with the above modified siRNAs. The results in Tables 1 and 2 are summarized, showing that the base-modified sequences siPRDX5-1 to siPRDX5-16 for siRNA of #3-siPRDX5, the base-modified sequences siGPX4-1 to siGPX4-16 for siRNA of #3-siGPX4, and the base-modified sequences siGPX4-(1) to siGPX4-(16) for siRNA of #4-siGPX4 all have potent interfering activity against drug-resistant or non-drug-resistant LNCaP cells and MyC-CaP cells, and the #3-siGPX4 is found to have superior interference activity than the #4-siGPX4. It is also found to be more efficient in interfering with human cells compared to mice.

**Table 1 Relative expression level of PRDX5 gene in different types of cells after the addition of siPRDX5**

| Cell type | #1-siPRDX5 | #2-siPRDX5 | #3-siPRDX5 | #3-siPRDX5-(NO.) |
|---|---|---|---|---|
| LNCaP | 0.605 | 0.580 | 0.125 | 0.067 |
| LNCaP-DTP | 0.621 | 0.629 | 0.197 | 0.102 |
| MyC-CaP | / | / | / | 0.3 |
| MyC-CaP-DTP | / | / | / | 0.298 |

| | | | | |
|---|---|---|---|---|
| Note: "NO." indicates the number of the base-modified sequence. | | | | |

**Table 2 Relative expression level of GPX4 gene in different types of cells after the addition of siGPX4**

| Cell type | #1-siGPX4 | #2-siGPX4 | #3-siGPX4 | #4-siGPX4 | #3-siGPX4-(NO.) | #4-siGPX4-(NO.) |
|---|---|---|---|---|---|---|
| LNCaP | 0.700 | 0.730 | 0.180 | 0.220 | 0.049 | 0.158 |
| LNCaP-DTP | 0.735 | 0.755 | 0.245 | 0.265 | 0.077 | 0.195 |
| MyC-CaP | / | / | / | / | 0.460 | / |
| MyC-CaP-DTP | / | / | / | / | 0.499 | / |

| | | | | | | |
|---|---|---|---|---|---|---|
| Note: "NO." indicates the number of the base-modified sequence. | | | | | | |

### Example 4 Killing ability of siPRDX5 and siGPX4 to drug-tolerant persister cells of prostate cancer

Further, drug-resistant LNCaP-DTP and MyC-CaP-DTP cells as well as 22Rv1 cells which are themselves ENZ-resistant were transfected with the modified #3-siPRDX5-1-16 or #3-siGPX4-1-16 or #3-siPRDX5-1-16 + #3-siGPX4-1-16 synthesized in Example 3 through a CCK8 method, illustrating the *in vitro* antitumor effects of nucleic acid drugs in drug-resistant cells.

### 1. Experimental method

The drug-resistant LNCaP-DTP and MyC-CaP-DTP cells as well as 22Rv1 cells which are themselves ENZ-resistant were seeded in a 96-well plate containing fresh 1640 medium and attached. Subsequently, 1 to 16 siPRDX5, siGPX4, siPRDX5+siGPX4, and negative control sineg were added, and the transfection mixture was sucked out after successful interference. The medium was replaced with a normal complete medium (1640 medium containing 10% fetal bovine serum and 1% double antibody) and continued to be cultured in the incubator for 24 h. Finally, a CCK8 assay was performed for determination and the cell survival was calculated by OD₄₅₀ value.

2. The results are shown in FIG. 5. FIG. 5A is a histogram showing the relative survival of drug-resistant LNCaP-DTP cells in human prostate cancer after administrating with 1 to 16 siRNAs of modified siPRDX5, respectively; FIG. 5B is a histogram showing the relative survival of drug-resistant MyC-CaP-DTP in human prostate cancer after administrating with 1 to 16 siRNAs of siPRDX5, respectively; FIG. 5C is a histogram showing the relative survival of ENZ-resistant 22Rv1 cells in human prostate cancer after administrating with 1 to 16 siRNAs of siPRDX5, respectively; FIG. 5D is a histogram showing the relative survival of drug-resistant LNCaP-DTP cells in human prostate cancer after administrating with 1 to 16 siRNAs of siGPX4, respectively; FIG. 5E is a histogram showing the relative survival of drug-resistant MyC-CaP-DTP cells in human prostate cancer after administrating with 1 to 16 siRNAs of siGPX4, respectively; FIG. 5F is a histogram showing the relative survival of ENZ-resistant 22Rv1 cells in human prostate cancer after administrating with 1 to 16 siRNAs of siGPX4, respectively; FIG. 5G is a histogram showing the relative survival of drug-resistant LNCaP-DTP cells in human prostate cancer after administrating with 1 to 16 siRNAs of siGPX4 combined one by one with 1 to 16 siRNAs of siPRDX5, respectively; FIG. 5H is a histogram showing the relative survival of drug-resistant MyC-CaP-DTP cells in human prostate cancer after administrating with 1 to 16 siRNAs of siGPX4 combined one by one with 1 to 16 siRNAs of siPRDX5, respectively; and FIG. 5I is a histogram showing the relative survival of ENZ-resistant 22Rv1 cells in human prostate cancer after administrating with 1 to 16 siRNAs of siGPX4 combined one by one with 1 to 16 siRNAs of siPRDX5, respectively.

The results show that in the drug-resistant DTP cells produced by continuous administration of ENZ for 9 days, the continued addition of ENZ no longer has a significant inhibitory effect. After the addition of different sequences of nucleic acid drugs siPRDX5 or siGPX4, about 50% inhibition rate can be reached for all three types of cells. And the addition of siPRDX5+siGPX4 inhibits the growth of the three cells more strongly than the addition of one siRNA alone. It indicates that siPRDX5 and siGPX4 have an obvious killing effect on drug-resistant cells in prostate cancer and have the ability to inhibit the growth of drug-resistant cells, and the effect realized by combining these two siRNAs is superior.

### Example 5 Effect of co-administration of ENZ and nucleic acid drugs on resistant-CRPC formed by continuous administration of ENZ in C-MYC-overexpressing prostate cancer mice model

The effect of co-administration of ENZ and modified #3-siPRDX5 or #3-siGPX4 on recurrent mice after chemical castration (i.e., continuous administration of ENZ) in a prostate cancer mouse model was further illustrated.

### 1. Experimental method

A *C-MYC* (Hi-Myc)-overexpressing and spontaneous prostate cancer mouse model was constructed. mPIN/Cancer transition was developed in mice at the age of 4 months, at which time the mice were randomly grouped into NC control group (gavaged with solvent) and ENZ administration group. The ENZ administration group was given 10 mg/Kg ENZ by gavage every three days for a total of 30 days, and then some of the mice were sacrificed by cervical dislocation and their prostates were taken for photographing and weighing. It was found that ENZ could significantly alleviate the condition, and the prostate weight was reduced by half compared to that of the NC control group. Afterwards, the rest of the mice continued to be administrated according to the above method for 60 days. A recurrence was found in the ENZ group, and the CRPC mice were successfully obtained. Afterwards, the mice (i.e., mice at the age of 7 months) were randomly grouped into NC control group (continuously gavaged with solvent), ENZ single drug group, nucleic acid drug siPRDX5-1 group, ENZ and siPRDX5-1 co-administration group, nucleic acid drug siPRDX5-16 group, ENZ and siPRDX5-16 co-administration group, nucleic acid drug siGPX4-1 group, ENZ and siGPX4-1 co-administration group, nucleic acid drug siGPX4-16 group, ENZ and siGPX4-16 co-administration group, nucleic acid drugs siPRDX5-16 + siGPX4-16 group, and ENZ and siPRDX5-16 + siGPX4-16 co-administration group. The corresponding administrations were performed. Except that NC and ENZ were still administrated by gavage, siPRDX5-1, siPRDX5-16, siGPX4-1, and siGPX4-16 were all administered by tail vein injection. The administrations were performed every three days for a total of 30 days, in which the dosage of ENZ was 10 mg/Kg, the dosage of siPRDX5-1/16 was 100 nM, and the dosage of siGPX4-1/16 was 100 nM. Afterwards, the mice were sacrificed by cervical dislocation and their prostates were taken for photographing, weighing and experiments such as HE staining.

2. The results are shown in FIG. 6 and FIG. 7. In FIG. 6, FIG. 6A is a graph showing the change in body weight of mice in each group in each group vs administration; FIG. 6B is a comparative graph showing the photographs of the prostate removed from mice in each group; and FIG. 6C is a graph showing the change in prostate weight of mice in each group vs administration. * indicates whether the reduction in prostate weights in other ENZ group and siPRDX5 and siGPX4 administration groups is significant compared with that in the NC group. In FIG. 7, FIG. 7A is a graph showing the HE staining of prostate tissue sections from mice in each group; and FIG. 7B is a histogram showing the relative changes in PRDX5 enzyme activity of prostate tissues from mice in each group. * indicates whether the reduction in PRDX5 enzyme activity in other siPRDX5 and siGPX4 administration groups is significant compared with that in the ENZ group.

The results for single administration and co-administration groups are shown in Table 3:

**Table 3 Effect of different administration (for 30 days) on drug resistance and recurrence**

| Administration | Mean mouse prostate weight |
|---|---|
| NC control group | 115.6 mg |
| Continuous ENZ single drug group after drug resistance and recurrence | 66.67 mg |
| nucleic acid drug siPRDX5-1 group after drug resistance and recurrence | 53.99 mg |
| ENZ and siPRDX5-1 co-administration group after drug resistance and recurrence | 49.95 mg |
| nucleic acid drug siPRDX5-16 group after drug resistance and recurrence | 49.58 mg |
| ENZ and siPRDX5-16 co-administration group after drug resistance and recurrence | 44.12 mg |
| nucleic acid drug siGPX4-1 group after drug resistance and recurrence | 57.81 mg |
| ENZ and siGPX4-1 co-administration group after drug resistance and recurrence | 49.40 mg |
| nucleic acid drug siGPX4-16 group after drug resistance and recurrence | 49.31 mg |
| ENZ and siGPX4-16 co-administration group after drug resistance and recurrence | 45.33 mg |
| nucleic acid drugs siPRDX5-16 + siGPX4-16 group after drug resistance and recurrence | 47.28 mg |
| ENZ and siPRDX5-16 + siGPX4-16 co-administration group after drug resistance and recurrence | 39.35 mg |

Combined with FIG. 6 and Table 3, it can be seen that the mean mouse prostate weight of the drug-resistant CRPC mice is 66.67 mg after continuous gavage of ENZ for 30 days, at which time this mean value of the NC control group is 115.6 mg, while the prostate weight of the siPRDX5-1 group after 30 days of administration has been reduced to 53.99 mg, and that of the siPRDX5-1 combined with ENZ group has been reduced to 49.95 mg, indicating that the nucleic acid drug siPRDX5 has a pharmacological efficacy in inhibiting tumor growth in CRPC mice. The prostate weight of the siGPX4-1 group after 30 days of administration is 57.81 mg, and that of the siGPX4-1 combined with ENZ group is 49.40 mg, indicating that the nucleic acid drug siGPX4 also has a pharmacological efficacy in inhibiting tumor growth in CRPC mice. The prostate weight of the siPRDX5-16 + siGPX4-16 group after 30 days of administration is reduced to 47.28 mg, and that of the siPRDX5-16 + siGPX4-16 and ENZ co-administration group is reduced to 39.35 mg, indicating that the efficacy of the nucleic acid drugs siPRDX5+siGPX4 is more significant.

The co-administration of ENZ and siPRDX5 or siGPX4 has a better efficacy than siPRDX5 or siGPX4 alone, and the prostate weight can be reduced to around 50 mg. It can also be seen that siPRDX5 or siGPX4 alone has a better therapeutic effect than ENZ alone in case of the drug-resistance, indicating that the inhibitory effect on the drug-resistant CRPC mice is achieved by siPRDX5 or siGPX4 alone. ENZ combined with two nucleic acid drugs has the best efficacy, and the prostate weight can be reduced to around 40 mg.

Assay for PRDX5 enzyme activity: the PRDX5 enzyme activity was determined using the thioredoxin system. Specifically, the rate of H₂O₂ degradation was measured by detecting the reduction of A340 caused by NADPH oxidation. Experiments were performed in a 150 µL reaction mixture containing 50 mM HEPES-NaOH (pH = 7), 200 µM NADPH, 760 nM mouse TXNRD1, 11 µM human TRX, and different concentration gradients of PRDX5. The incubation was performed at 37°C for 5 min and the reaction was initiated by the addition of 500 µM H₂O₂.

The results of PRDX5 enzyme activity in prostate of different groups are shown in Table 4:

**Table 4 Effect of different administration (for 30 days) on PRDX5 enzyme activity in prostate**

| Administration | PRDX5 enzyme activity relative to NC group |
|---|---|
| NC control group | 1 |
| Continuous ENZ single drug group after drug resistance and recurrence | 4.560 |
| nucleic acid drug siPRDX5-1 group after drug resistance and recurrence | 2.025 |
| ENZ and siPRDX5-1 co-administration group after drug resistance and recurrence | 1.465 |
| nucleic acid drug siPRDX5-16 group after drug resistance and recurrence | 1.800 |
| ENZ and siPRDX5-16 co-administration group after drug resistance and recurrence | 1.290 |
| nucleic acid drug siGPX4-1 group after drug resistance and recurrence | 1.810 |
| ENZ and siGPX4-1 co-administration group after drug resistance and recurrence | 1.600 |
| nucleic acid drug siGPX4-16 group after drug resistance and recurrence | 1.715 |
| ENZ and siGPX4-16 co-administration group after drug resistance and recurrence | 1.255 |
| nucleic acid drugs siPRDX5-16 + siGPX4-16 group after drug resistance and recurrence | 1.350 |
| ENZ and siPRDX5-16 + siGPX4-16 co-administration group after drug resistance and recurrence | 0.955 |

From the results of HE staining of tissue sections (FIG. 7), it can be seen that there is significant killing, degeneration and fibrosis of prostate tumors of CRPC after the co-administration. Combined with the results of PRDX5 enzyme activity in the prostate of different groups (Table 4), it can be seen that the PRDX5 enzyme activity in mice with CRPC formed by continuous gavage of ENZ increases significantly, reaching a relative value of 4.56, and the enzyme activity can be significantly inhibited with the nucleic acid drug siPRDX5-1, reaching a relative value of 2.025, especially when the co-administration is performed, a relative value of 1.465 can be reached. After administrating the nucleic acid drug siGPX4-1, the PRDX5 enzyme activity can also be significantly inhibited, reaching a relative value of 1.810, and when the co-administration is performed, a relative value of 1.600 can be reached. It demonstrates that the two nucleic acid drugs have significant interference effects when they are used alone, respectively. And when the two nucleic acid drugs are co-administered, the relative enzyme activity value of PRDX5 in the siPRDX5-16 + siGPX4-16 group reaches 1.350, and the enzyme activity can reach the lowest value of 0.955 when co-administered with ENZ, indicating that the co-administration of the nucleic acid drugs siPRDX5-16+siGPX4-16 can reach the optimal inhibition of the PRDX5 enzyme activity, and the optimal efficacy of inhibiting prostate tumor growth in CRPC mice.

Although the present disclosure has been disclosed as above in preferred examples, it is not intended to limit the present disclosure, and those skilled in the art may make various changes and modifications without departing from the spirit and scope according to the present disclosure. Therefore, the scope of protection of the present disclosure should be based on that defined in the claims.

## Claims

1. A double-stranded siRNA molecule, as shown in any one of the following (A) to (C):
(A) an siRNA molecule that inhibits the expression of a PRDX5 gene, having a double-stranded siRNA molecule formed by an RNA single strand set forth in SEQ ID NO. 1 and an RNA single strand set forth in SEQ ID NO. 2 which are complementary to each other;
(B) an siRNA molecule that inhibits the expression of a GPX4 gene, having a double-stranded siRNA molecule formed by an RNA single strand set forth in SEQ ID NO. 3 and an RNA single strand set forth in SEQ ID NO. 4 which are complementary to each other; or having a double-stranded siRNA molecule formed by an RNA single strand set forth in SEQ ID NO. 5 and an RNA single strand set forth in SEQ ID NO. 6 which are complementary to each other;
(C) a composition of an siRNA molecule that inhibits the expression of a GPX4 gene and (A), the siRNA molecule that inhibits the expression of the GPX4 gene having a double-stranded siRNA molecule formed by an RNA single strand set forth in SEQ ID NO. 3 and an RNA single strand set forth in SEQ ID NO. 4 which are complementary to each other.

2. The double-stranded siRNA molecule according to claim 1, wherein at least one nucleotide in the double-stranded siRNA molecule is a modified nucleotide.

3. The double-stranded siRNA molecule according to claim 1 or 2, wherein all of the nucleotides in the double-stranded siRNA molecule are modified nucleotides.

4. The double-stranded siRNA molecule according to claim 2 or 3, wherein the modification is selected from at least one of phosphorothioate, 2'-F, 2'-OMe, 2'-Ara-F, 2'-O-MO, m⁶A or m⁵C.

5. The double-stranded siRNA molecule according to any one of claims 2-4, as shown in any one of the following (A1) to (C16):
(A1)
5' (C)-[A]-(G)[A](C)[U](U)[A](UUA)[C](U)[A](G)[A](U)[G](A)[U](T) 3';
5' [A]-(A)-[U](C)[A](U)[C](U)[A](G)[UAA](U)[A](A)[G](U)[C](U)[G]-[U]-[C] 3';
(A2)
5' (C)-[A]-(G)[A](C)[U](U)[A](UUA)[C](U)[A](G)[A](U)[G](A)[U](T) 3';
5' [A]-(A)-[U](C)[A](U)[C](U)[A](G)[UAA](U)[A](A)[G](U)[C](U)[G]-[U]-[C] 3';
(A3)
5' (C)-[A]-(G)[A](C)[U](U)[A](UUA)[C](U)[A](G)[A](U)[G](A)[U](T) 3';
5' [A]-(A)-[U](C)[A](U)[C](U)[A](G)[UAA](U)[A](A)[G](U)[C](U)[G]-[U]-[C] 3';
(A4)
5' (C)-[A]-(G)[A](C)[U](U)[A](UUA)[C](U)[A](G)[A](U)[G](A)[U](T) 3';
5' [A]-(A)-[U](C)[A](U)[C](U)[A](G)[UAA](U)[A](A)[G](U)[G](U)[G]-[U]-[C] 3';
(A5)
5' (C)-[A*]-(G)[A*](C)[U](U)[A*](UUA*)[C](U)[A*](G)[A*](U)[G](A*)[U](T) 3';
5' [A*]-(A*)-[U](C)[A*](U)[C](U)[A*](G)[UA*A*](U)[A*](A*)[G](U)[C](U)[G]-[U]-[C] 3';
(A6)
5' (C')-[A]-(G)[A](C')[U](U)[A](UUA)[C'](U)[A](G)[A](U)[G](A)[U](T) 3';
5' [A]-(A)-[U](C')[A](U)[C'](U)[A](G)[UAA](U)[A](A)[G](U)[C'](U)[G]-[U]-[C'] 3';
(A7)
5' (C)-[A*]-(G)[A*](C)[U](U)[A*](UUA*)[C](U)[A*](G)[A*](U)[G](A*)[U](T) 3';
5'[A*]-(A*)-[U](C)[A*](U)[C](U)[A*](G)[UA*A*](U)[A*](A*)[G](U)[C](U)[G]-[U]-[C] 3';
(A8)
5' (C)-[A*]-(G)[A*](C)[U](U)[A*](UUA*)[C](U)[A*](G)[A*](U)[G](A*)[U](T) 3';
5' [A*]-(A*)-[U](C)[A*](U)[C](U)[A*](G)[UA*A*](U)[A*](A*)[G](U)[C](U)[G]-[U]-[C] 3';
(A9)
5' (C)-[A*]-(G)[A*](C)[U](U)[A*](UUA*)[C](U)[A*](G)[A*](U)[G](A*)[U](T) 3';
5' [A*]-(A*)-[U](C)[A*](U)[C](U)[A*](G)[UA*A*](U)[A*](A*)[G](U)[C](U)[G]-[U]-[C] 3';
(A10)
5' (C')-[A]-(G)[A](C')[U](U)[A](UUA)[C'](U)[A](G)[A](U)[G](A)[U](T) 3';
5' [A]-(A)-[U](C')[A](U)[C'](U)[A](G)[UAA](U)[A](A)[G](U)[C'](U)[G]-[U]-[C'] 3';
(A11)
5' (C')-[A]-(G)[A](C')[U](U)[A](UUA)[C'](U)[A](G)[A](U)[G](A)[U](T) 3';
5' [A]-(A)-[U](C')[A](U)[C'](U)[A](G)[UAA](U)[A](A)[G](U)[C'](U)[G]-[U]-[C'] 3';
(A12)
5' (C')-[A]-(G)[A](C')[U](U)[A](UUA)[C'](U)[A](G)[A](U)[G](A)[U](T) 3';
5' [A]-(A)-[U](C')[A](U)[C'](U)[A](G)[UAA](U)[A](A)[G](U)[C'](U)[G]-[U]-[C'] 3';
(A13)
5' (C')-[A*]-(G)[A*](C')[U](U)[A*](UUA*)[C'](U)[A*](G)[A*](U)[G](A*)[U](T) 3';
5'[A*]-(A*)-[U](C')[A*](U)[C'](U)[A*](G)[UA*A*](U)[A*](A*)[G](U)[C'](U)[G]-[U]-[C']3';
(A14)
5' (C')-[A*]-(G)[A*](C')[U](U)[A*](UUA*)[C'](U)[A*](G)[A*](U)[G](A*)[U](T) 3';
5'[A*]-(A*)-[U](C')[A*](U[C'](U)[A*](G)[UA*A*](U)[A*](A*)[G](U)[C'](U)[G]-[U]-[C']3';
(A15)
5' (C')-[A*]-(G)[A*](C')[U](U)[A*](UUA*)[C'](U)[A*](G)[A*](U)[G](A*)[U](T) 3';
5'[A*]-(A*)-[U](C')[A*](U)[C'](U)[A*](G)[UA*A*](U)[A*](A*)[G](U)[C'](U)[G]-[U]-[C']3';
(A16)
5' (C')-[A*]-(G)[A*](C')[U](U)[A*](UUA*)[G'](U)[A*](G)[A*](U)[G](A*)[U](T) 3';
5'[A*]-(A*)-[U](C')[A*](U[C'](U)[A*](G)[UA*A*](U)[A*](A*)[G](U)[C'](U)[G]-[U]-[C']3';
(B1)
5' (A)-[C]-(A)[A](C)[G](U)[C](AAA)[U](U)[C](G)[A](U)[A](U)[G](U) 3';
5' [A]-(C)-[A](U)[A](U)[C](G)[A](A)[UUU](G)[A](C)[G](U)[U](G)[U]-[A]-[C] 3';
(B2)
5' (A)-[C]-(A)[A](C)[G](U)[C](AAA)[U](U)[C](G)[A](U)[A](U)[G](U) 3';
5' [A]-(C)-[A](U)[A](U)[C](G)[A](A)[UUU](G)[A](C)[G](U)[U](G)[U]-[A]-[C] 3';
(B3)
5' (A)-[C]-(A)[A](C)[G](U)[C](AAA)[U](U)[C](G)[A](U)[A](U)[G](U) 3';
5' [A]-(C)-[A](U)[A](U)[C](G)[A](A)[UUU](G)[A](C)[G](U)[U](G)[U]-[A]-[C] 3';
(B4)
5' (A)-[C]-(A)[A](C)[G](U)[C](AAA)[U](U)[C](G)[A](U)[A](U)[G](U) 3';
5' [A]-(C)-[A](U)[A](U)[C](G)[A](A)[UUU](G)[A](C)[G](U)[U](G)[U]-[A]-[C] 3';
(B5)
5' (A*)-[C]-(A*)[A*](C)[G](U)[C](A*A*A*)[U](U)[C](G)[A*](U)[A*](U)[G](U) 3';
5' [A*]-(C)-[A*](U)[A*](U)[C](G)[A*](A*)[UUU](G)[A*](C)[G](U)[U](G)[U]-[A*]-[C] 3';
(B6)
5' (A)-[C']-(A)[A](C')[G](U)[C'](AAA)[U](U)[C'](G)[A](U)[A](U)[G](U) 3';
5' [A]-(C')-[A](U)[A](U)[C'](G)[A](A)[UUU](G)[A](C')[G](U)[U](G)[U]-[A]-[C'] 3';
(B7)
5' (A*)-[C]-(A*)[A*](C)[G](U)[C](A*A*A*)[U](U)[C](G)[A*](U)[A*](U)[G](U) 3';
5' [A*]-(C)-[A*](U)[A*](U)[C](G)[A*](A*)[UUU](G)[A*](C)[G](U)[U](G)[U]-[A*]-[C] 3';
(B8)
5' (A*)-[C]-(A*)[A*](C)[G](U)[C](A*A*A*)[U](U)[C](G)[A*](U)[A*](U)[G](U) 3';
5' [A*]-(C)-[A*](U)[A*](U)[C](G)[A*](A*)[UUU](G)[A*](C)[G](U)[U](G)[U]-[A*]-[C] 3';
(B9)
5' (A*)-[C]-(A*)[A*](C)[G](U)[C](A*A*A*)[U](U)[C](G)[A*](U)[A*](U)[G](U) 3';
5' [A*]-(C)-[A*](U)[A*](U)[C](G)[A*](A*)[UUU](G)[A*](C)[G](U)[U](G)[U]-[A*]-[C] 3';
(B10)
5' (A)-[C']-(A)[A](C')[G](U)[C'](AAA)[U](U)[C'](G)[A](U)[A](U)[G](U) 3';
5' [A]-(C')-[A](U)[A](U)[C'](G)[A](A)[UUU](G)[A](C')[G](U)[U](G)[U]-[A]-[C'] 3';
(B11)
5' (A)-[C']-(A)[A][C'](G)(U)[C'](AAA)[U](U)[C'](G)[A](U)[A](U)[G](U) 3';
5' [A]-(C')-[A](U)[A](U)[C'](G)[A](A)[UUU](G)[A](C')[G](U)[U](G)[U]-[A]-[C'] 3';
(B12)
5' (A)-[C']-(A)[A](C')[G](U)[C'](AAA)[U](U)[C'](G)[A](U)[A](U)[G](U) 3';
5' [A]-(C')-[A](U)[A](U)[C'](G)[A](A)[UUU](G)[A](C')[G](U)[U](G)[U]-[A]-[C'] 3';
(B13)
5' (A*)-[C']-(A*)[A*](C')[G](U)[C'](A*A*A*)[U](U)[C'](G)[A*](U)[A*](U)[G](U) 3';
5' [A*]-(C')-[A*](U)[A*](U)[C'](G)[A*](A*)[UUU](G)[A*](C')[G](U)[U](G)[U]-[A*]-[C'] 3';
(B14)
5' (A*)-[C']-(A*)[A*](C')[G](U)[C'](A*A*A*)[U](U)[C'](G)[A*](U)[A*](U)[G](U) 3';
5' [A*]-(C')-[A*](U)[A*](U)[C'](G)[A*](A*)[UUU](G)[A*](C')[G](U)[U](G)[U]-[A*]-[C'] 3';
(B15)
5' (A*)-[C']-(A*)[A*](C')[G](U)[C'](A*A*A*)[U](U)[C'](G)[A*](U)[A*](U)[G](U) 3';
5' [A*]-(C')-[A*](U)[A*](U)[C'](G)[A*](A*)[UUU](G)[A*](C')[G](U)[U](G)[U]-[A*]-[C'] 3';
(B16)
5' (A*)-[C']-(A*)[A*](C')[G](U)[C'](A*A**)[U](U)[C'](G)[A*](U)[A*](U)[G](U) 3';
5' [A*]-(C')-[A*](U)[A*](U)[C'](G)[A*](A*)[UUU](G)[A*](C')[G](U)[U](G)[U]-[A*]-[C'] 3';
(C1)
5' (G)-[U]-(G)[A](G)[G](C)[A](AGA)[C](C)[G](A)[A](G)[U](A)[A](A) 3';
5' [U]-(U)-[U](A)[C](U)[U](C)[G](G)[UCU](U)[G](C)[C](U)[C](A)[C]-[U]-[G] 3';
(C2)
5' (G)-[U]-(G)[A](G)[G](C)[A](AGA)[C](C)[G](A)[A](G)[U](A)[A](A) 3';
5' [U]-(U)-[U](A)[C](U)[U](C)[G](G)[UCU](U)[G](C)[C](U)[C](A)[C]-[U]-[G] 3';
(C3)
5' (G)-[U]-(G)[A](G)[G](C)[A](AGA)[C](C)[G](A)[A](G)[U](A)[A](A) 3';
5' [U]-(U)-[U](A)[C](U)[U](C)[G](G)[UCU](U)[G](C)[C](U)[C](A)[C]-[U]-[G] 3';
(C4)
5' (G)-[U]-(G)[A](G)[G](C)[A](AGA)[C](C)[G](A)[A](G)[U](A)[A](A) 3';
5' [U]-(U)-[U](A)[C](U)[U](C)[G](G)[UCU](U)[G](C)[C](U)[C](A)[C]-[U]-[G] 3';
(C5)
5' (G)-[U]-(G)[A*](G)[G](C)[A*](A*GA*)[C](C)[G](A*)[A*](G)[U](A*)[A*](A*) 3';
5' [U]-(U)-[U](A*)[C](U)[U](C)[G](G)[UCU](U)[G](C)[C](U)[C](A*)[C]-[U]-[G] 3';
(C6)
5' (G)-[U]-(G)[A](G)[G](C')[A](AGA)[C'](C')[G](A)[A](G)[U](A)[A](A) 3';
5' [U]-(U)-[U](A)[C'](U)[U](C')[G](G)[UC'U](U)[G](C')[C'](U)[C'](A)[C']-[U]-[G] 3';
(C7)
5' (G)-[U]-(G)[A*](G)[G](C)[A*](A*GA*)[C](C)[G](A*)[A*](G)[U](A*)[A*](A*) 3';
5' [U]-(U)-[U](A*)[C](U)[U](C)[G](G)[UCU](U)[G](C)[C](U)[C](A*)[C]-[U]-[G] 3';
(C8)
5' (G)-[U]-(G)[A*](G)[G](C)[A*](A*GA*)[C](C)[G](A*)[A*](G)[U](A*)[A*](A*) 3';
5' [U]-(U)-[U](A*)[C](U)[U](C)[G](G)[UCU](U)[G](C)[C](U)[C](A*)[C]-[U]-[G] 3';
(C9)
5' (G)-[U]-(G)[A*](G)[G](C)[A*](A*QA*)[C](C)[G](A*)[A*](G)[U](A*)[A*](A*) 3';
5' [U]-(U)-[U](A*)[C](U)[U](C)[G](G)[UCU](U)[G](C)[C](U)[C](A*)[C]-[U]-[G] 3';
(C10)
5' (G)-[U]-(G)[A](G)[G](C')[A](AGA)[C'](C')[G](A)[A](G)[U](A)[A](A) 3';
5' [U]-(U)-[U](A)[C'](U)[U](C')[G](G)[UC'U](U)[G](C')[C'](U)[C'](A)[C']-[U]-[G] 3';
(C11)
5' (G)-[U]-(G)[A](G)[G](C')[A](AGA)[C'](C')[G](A)[A](G)[U](A)[A](A) 3';
5' [U]-(U)-[U](A)[C'](U)[U](C')[G](G)[UC'U](U)[G](C')[C'](U)[C'](A)[C']-[U]-[G] 3';
(C12)
5' (G)-[U]-(G)[A](G)[G](G')[A](AGA)[C'](C')[G](A)[A](G)[U](A)[A](A) 3';
5' [U]-(U)-[U](A)[C'](U)[U](C')[G](G)[UC'U](U)[G](C')[C'](U)[G'](A)[C']-[U]-[G] 3';
(C13)
5' (G)-[U]-(G)[A*](G)[G](C')[A*](A*GA*)[C'](C')[G](A*)[A*](G)[U](A*)[A*](A*) 3';
5' [U]-(U)-[U](A*)[C'](U)[U](C')[G](G)[UC'U](U)[G](C')[C'](U)[C'](A*)[C']-[U]-[G] 3';
(C14)
5' (G)-[U]-(G)[A*](G)[G](C')[A*](A*GA*)[C'](C')[G](A*)[A*](G)[U](A*)[A*](A*) 3';
5' [U]-(U)-[U](A*)[C'](U)[U](C')[G](G)[UC'U](U)[G](C')[C'](U)[C'](A*)[C']-[U]-[G] 3';
(C15)
5' (G)-[U]-(G)[A*](G)[G](C')[A*](A*GA*)[C'](C')[G](A*)[A*](G)[U](A*)[A*](A*) 3';
5' [U]-(U)-[U](A*)[C'](U)[U](C')[G](G)[UC'U](U)[G](C')[C'](U)[C'](A*)[C']-[U]-[G] 3';
(C16)
5' (G)-[U]-(G)[A*](G)[G](C')[A*](A*GA*)[C'](C')[G](A*)[A*](G)[U](A*)[A*](A*) 3';
5' [U]-(U)-[U](A*)[C'](U)[U](C')[G](G)[UC'U](U)[G](C')[C'](U)[C'](A*)[C']-[U]-[G] 3';
wherein A-, U-, C-, and G- denote phosphorothioate-modified ribonucleotides A, U, C, and G, respectively;
(A), (U), (C), and (G) denote 2'-F-modified ribonucleotides A, U, C, and G, respectively;
[A], [U], [C] and [G] denote 2'-OMe-modified ribonucleotides A, U, C and G, respectively;
(A), (U), (C) and (G) denote 2'-Ara-F-modified ribonucleotides A, U, C and G, respectively;
[A], [U], [C] and [G] denote 2'-O-MO-modified ribonucleotides A, U, C and G, respectively;
A*, U*, C* and G* denote m⁶A-modified ribonucleotides A, U, C and G, respectively; and
A', U', C' and G' denote m⁵C-modified ribonucleotides A, U, C and G, respectively.

6. A biological material associated with the siRNA according to any one of claims 1-5, being any one of the following:
(1) a vector expressing the siRNA;
(2) a host cell carrying the siRNA or the vector;
(3) a reagent containing the siRNA, or the vector in (1) or the host cell in (2); and
(4) a pharmaceutical composition containing the siRNA.

7. The biological material according to claim 6, wherein the pharmaceutical composition may further contain an androgen or an androgen receptor inhibitor.

8. The biological material according to claim 6 or 7, wherein the pharmaceutical composition contains a pharmaceutically acceptable carrier or excipient.

9. The biological material according to claim 8, wherein the pharmaceutically acceptable carrier comprises a microliposome, a microcell, a metal particle, or a polymer particle.

10. The application of the siRNA according to claim 1~5 or the biological material according to claim 6~9 in preparation of drugs for the prevention or treatment of a prostate cancer.

11. The application according to claim 10, wherein the prostate cancer is an advanced prostate cancer or castration-resistant prostate cancer.

12. A method for preventing or treating prostate cancer, comprising administrating the siRNA according to any one of claims 1-5 or the biological material according to any one of claims 6-9 to a subject.

13. A method for inhibiting the expression of PRDX5 and/or GPX4 genes in cells, comprising contacting the cells with an effective amount of the siRNA according to any one of claims 1 to 5, or the biological material according to any one of claims 6 to 9, thereby inhibiting the expression of PRDX5 and/or GPX4 genes in the cells.
